(19) Europäisches Patentamt
European Patent Office
Office européen des brevets

(11) EP 1 751 318 B1

(12) **EUROPÄISCHE PATENTSCHRIFT**

(45) Veröffentlichungstag und Bekanntmachung des Hinweises auf die Patenterteilung:
**05.01.2011 Patentblatt 2011/01**

(21) Anmeldenummer: **05752803.6**

(22) Anmeldetag: **13.05.2005**

(51) Int Cl.:
***C14C 9/02*** *(2006.01)* ***C07C 43/11*** *(2006.01)*
***C08G 71/00*** *(2006.01)*

(86) Internationale Anmeldenummer:
**PCT/EP2005/005225**

(87) Internationale Veröffentlichungsnummer:
**WO 2005/113839 (01.12.2005 Gazette 2005/48)**

(54) **WÄSSRIGE ZUSAMMENSETZUNGEN, ENTHALTEND ALKOXYLIERTE ALKOHOLE UND HYDROPHOBE KOMPONENTE, VERFAHREN ZU IHRER HERSTELLUNG UND IHRE VERWENDUNG**

AQUEOUS COMPOSITIONS CONTAINING ALKOXYLATED ALCOHOLS AND HYDROPHOBIC COMPONENTS, METHOD FOR THE PRODUCTION AND USE THEREOF

COMPOSITIONS, LEUR PROCEDE DE PRODUCTION ET LEUR UTILISATION

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **17.05.2004 DE 102004024798**

(43) Veröffentlichungstag der Anmeldung:
**14.02.2007 Patentblatt 2007/07**

(73) Patentinhaber: **BASF SE**
**67056 Ludwigshafen (DE)**

(72) Erfinder:
- **ERHARDT, Rainer**
**68199 Mannheim (DE)**
- **PABST, Gunther**
**92318 Neumarkt (DE)**
- **OETTER, Günter**
**67227 Frankenthal (DE)**
- **SEITZ, Andreas**
**67134 Birkenheide (DE)**
- **DANISCH, Peter**
**67065 Ludwigshafen (DE)**

(56) Entgegenhaltungen:
**WO-A-03/023069** **DE-A1- 10 242 401**
**DE-A1- 19 740 452** **US-B1- 6 610 751**



Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Erfindung betrifft Zusammensetzungen, enthaltend als Komponenten

(a) mindestens zwei verschiedene alkoxylierte Alkohole,
(b) mindestens eine hydrophobe Komponente, gewählt aus natürlichen, modifizierten natürlichen und synthetischen hydrophoben Stoffen,
(c) mindestens 20 Gew.-%, bezogen auf das Gesamtgewicht der Zusammensetzung, an Wasser,
dadurch gekennzeichnet, dass die Zusammensetzungen einen mittleren hydrodynamischen Radius im Bereich von 1 bis 200 nm aufweisen, bestimmt durch dynamische Lichtstreuung,
und dass die Zusammensetzungen in wässriger Verdünnung von 1 : 499 (Volumenanteile) eine Transmission von mindestens 80% bei einer Wellenlänge von 400 nm und einer Probenschichtdicke von 1 cm aufweisen.

Weiterhin betrifft die vorliegende Erfindung Verfahren zur Herstellung der erfindungsgemäßen Zusammensetzungen und Verwendungen der erfindungsgemäßen Zusammensetzungen.
Emulgierbare Fettungsmittel, auch Licker genannt, spielen bei der Herstellung von Leder und Pelzfellen eine wichtige Rolle. Fettungsmittel dienen zum Weichmachen des Leders bzw. der Pelzfelle, zur Steigerung ihrer Fülle und zur Erhöhung der Schutzwirkung gegen Nässe, Schmutz und unerwünschte chemische Einflüsse. Eine Übersicht über klassische Fettungsmittel findet sich bei Herfeld, "Bibliothek des Leders", Bd. 4, S. 13 (1987). Handelsübliche Fettungsmittel bestehen üblicherweise aus chemisch modifizierten nativen Fetten, fetten Ölen, Wachsen, Harzen und Derivaten, Erdölfraktionen oder deren Folgeprodukten, vgl. Herfeld, "Bibliothek des Leders", Bd. 4, S. 59 (1987).
Ein wesentliches Problem ist bei vielen Fettungsmittel die Lagerstabilität. Die meisten Fettungsmittel werden in Form von Emulsionen verkauft, die sich nach einer gewissen Lagerzeit entmischen. Um die Lagerstabilität zu erhöhen und die Entmischung der Emulsionen zu verzögern, setzt man den Emulsionen Emulgatoren zu. Man beobachtet jedoch, dass in vielen Fällen durch Zusatz größerer Mengen an Emulgator die Auszehrung sinkt.
Ein weiteres wesentliches Problem ist die in vielen Fällen noch verbesserungsfähige Auszehrung. Leder bzw. Pelzfelle werden üblicherweise in wässrigen Flotten mit Fettungsmitteln behandelt. Nach der Fettung werden die wässrigen Flotten üblicherweise entsorgt, und nicht verbrauchtes Fettungsmittel muss in Kläranlagen abgebaut werden und verursacht einen erhöhten Chemischen Sauerstoffbedarf (CSB) der Flotten.
Es hat sich weiterhin gezeigt, dass zahlreiche lagerstabile Fettungsmittel zunächst sehr gut fetten, aber nach einiger Zeit zu unerwünschten sogenannten Fettausschlägen führen. Diese Fettausschläge geben dem Leder ein unvorteilhaft fleckiges Aussehen.
Gewünscht ist weiterhin, dass Fettungsmittel einen geringen Anteil an flüchtigen organischen Verbindungen aufweisen (geringer VOC) und bei der Verwendung zur Herstellung von beispielsweise Autoleder ein nur geringes Fogging-Problem verursachen. DIN 75201 bzw. DIN 75201B definieren Fogging als Kondensation von verdampften flüchtigen Bestandteilen aus der Fahrzeuginnenausstattung an den Glasscheiben, insbesondere an der Windschutzscheibe.
Aus WO 03/23069 ist bekannt, dass gewisse Kombinationen von alkoxylierten Alkanolen bzw. Fettalkoholen sehr gute Emulgatoren sind und zur von Fettung von Leder geeignet sind. WO 03/23069 offenbart jedoch keine lagerstabilen Fettungsmittel.
DE 197 40 452 offenbart Mikroemulsionen, die $C_{12}$-$C_{22}$-Fettsäuremethylester, Sulfobernsteinsäureester und nichtionische Emulgatoren in bestimmten Mengenverhältnissen enthalten, sowie ihre Verwendung als Gleit- oder Glättemittel für die Faserbehandlung.
Es bestand also die Aufgabe, Zusammensetzungen bereit zu stellen, welche die oben genannten Nachteile nicht aufweisen
Demgemäß wurden eingangs definierte Zusammensetzungen gefunden.
(a), (b), (c), (d) und (e) werden im Folgenden auch als Komponente (a), Komponente (b) usw. bezeichnet.
Erfindungsgemäße Zusammensetzungen enthalten als Komponente (a) mindestens zwei, bevorzugt eine Mischung von mindestens zwei, bevorzugt mindestens 3 verschiedenen alkoxylierten Alkoholen. Vorzugsweise enthalten erfindungsgemäße Zusammensetzungen als Komponente (a) Mischungen von mindestens zwei, insbesondere mindestens drei alkoxylierten Alkoholen (a1), (a2) und (a3). Besonders bevorzugt enthalten erfindungsgemäße Zusammensetzungen mindestens einen alkoxylierten Alkohol (a1), mindestens einen alkoxylierten Alkohol (a2) und mindestens einen alkoxylierten Alkohol (a3).
Alkoxylierte Alkohole (a1) werden gewählt aus $C_6$-$C_{14}$-Alkanolen, bevorzugt aus $C_8$-$C_{12}$-Alkanolen, besonders bevorzugt $C_{10}$-Alkanolen, jeweils alkoxyliert mit im Mittel (Zahlenmittel) 4 bis 12 Äquivalenten Alkylenoxid, bevorzugt im Mittel 5 bis 10 Äquivalenten Alkylenoxid.
Alkoxylierte Alkohole (a2) werden gewählt aus einem oder mehreren $C_{12}$-$C_{24}$-Alkanolen, bevorzugt einem oder mehreren $C_{14}$-$C_{20}$-Alkanolen, besonders bevorzugt einem oder mehreren $C_{16}$-$C_{18}$-Alkanolen, der alkoxyliert ist bzw.

die alkoxyliert sind mit im Mittel (Zahlenmittel) 15 bis 40 Äquivalenten Alkylenoxid, bevorzugt 20 bis 30 Äquivalenten Alkylenoxid.

Alkoxylierte Alkohole (a3) werden gewählt aus einem oder mehreren $C_{12}$-$C_{24}$-Alkanolen, bevorzugt einem oder mehreren $C_{14}$-$C_{20}$-Alkanolen, besonders bevorzugt einem oder mehreren $C_{16}$-$C_{18}$-Alkanolen, der alkoxyliert ist bzw. die alkoxyliert sind mit im Mittel (Zahlenmittel) 50 bis 100 Äquivalenten Alkylenoxid.

Vorzugsweise sind den alkoxylierten Alkoholen (a3) zugrundeliegenden $C_6$-$C_{14}$-Alkanole bzw. $C_{12}$-$C_{24}$-Alkanote jeweils n-Alkanole oder Gemische aus n-Alkanolen und iso-Alkanolen; besonders bevorzugt sind die den alkoxylierten Alkoholen (a3) zugrunde liegenden $C_{12}$-$C_{24}$-Alkanole jeweils n-Alkanole.

Unter Alkylenoxiden werden im Rahmen der vorliegenden Erfindung gleiche oder verschiedene $C_2$-$C_4$-Alkylenoxide, wie beispielsweise Butylenoxid, Propylenoxid und insbesondere Ethylenoxid verstanden. Wünscht man zur Herstellung von beispielsweise (a2) verschiedene Alkylenoxide einzusetzen, so können die Alkylenoxideinheiten in (a2) beispielsweise blockweise, alternierend und bevorzugt statistisch angeordnet sein.

Oben aufgeführte alkoxylierte Alkohole fallen aufgrund der Synthese üblicherweise in Form von Gemischen an, wobei sich die Komponenten der anfallenden Gemische üblicherweise durch ihren Alkoxylierungsgrad unterscheiden. Der Alkoxylierungsgrad bezeichnet daher im Rahmen der vorliegenden Erfindung den mittleren Alkoxylierungsgrad (Zahlenmittel), der sich durch dem Fachmann bekannte Methoden wie beispielsweise Gelpermeationschromatographie (GPC) bestimmen lässt. Ein durch eine übliche Synthese anfallendes Gemisch wird im Rahmen der vorliegenden Erfindung nicht als zwei verschiedene alkoxylierte Alkohole definiert.

Oben aufgeführte alkoxylierte Alkohole (a1), (a2) und (a3) sind als solche bekannt und können nach bekannten Verfahren durch Umsetzung des oder der betreffenden Alkohole mit den gewünschten Mengen an Alkylenoxid. Die Umsetzung kann man durch Zugabe von geringen Wasser und/oder Alkalimetallhydroxid katalysieren.

Komponente (a) kann man durch Zusammenrühren von mindestens zwei verschiedenen, insbesondere mindestens drei alkoxylierten Alkoholen (a1), (a2) und (a3) herstellen. Besonders bevorzugt enthalten erfindungsgemäße Zusammensetzungen mindestens einen alkoxylierten Alkohol (a1), mindestens einen alkoxylierten Alkohol (a2) und mindestens einen alkoxylierten Alkohol (a3).

Die relativen Anteile von (a1), (a2) und (3) kann man in beliebigen Grenzen wählen.

In einer bevorzugten Ausführungsform wählt man (a) wie folgt:

20 bis 60 Gew.-%, bevorzugt 25 bis 50 und besonders bevorzugt 28 bis 40 Gew.-% (a1),
20 bis 70 Gew.-%, bevorzugt 25 bis 60 Gew.-% und besonders bevorzugt 30 bis 45 Gew.-% (a2) und
10 bis 50 Gew.-%, bevorzugt 15 bis 40 Gew.-% und besonders bevorzugt 22 bis 32 Gew.-% (a3).

Erfindungsgemäße Zusammensetzungen enthalten weiterhin mindestens eine hydrophobe Komponente (b), die gewählt wird aus natürlichen, modifizierten natürlichen und synthetischen hydrophoben Stoffen.

Geeignete natürliche hydrophobe Stoffe sind beispielsweise natürliche Wachse wie beispielsweise Bienenwachs, Korkwachs, Montanwachse oder Carnaúbawachs, natürliche Triglyceride und native Öle und native Fette wie beispielsweise Stearin, Tungöl, Fischöl, Chaulmoograöl, Lanolin, Schellackwachs sowie deren Gemische, und insbesondere Knochenöl, Nitöl, Klauenöl wie beispielsweise Rinderklauenöl, Lardöl, Triolein, Rapsöl, Nussöl, Olivenöl und Ricinusöl.

Geeignete modifizierte natürliche hydrophobe Stoffe sind beispielsweise gewählt aus mit beispielsweise Methanol oder Ethanol veresterten Fettsäuren oder Fettsäuregemischen, insbesondere Rapsölmethylester; oxidierten und sulfitierten natürlichen Ölen und aus oxidierten und sulfatierten natürlichen Ölen, wobei oxidierte und sulfitierte Öle bevorzugt sind. Besonders bevorzugt sind oxidiertes und sulfitiertes Rapsöl und oxidiertes und sulfitiertes Fischöl und Mischungen der vorstehend genannten modifizierten hydrophoben Stoffe.

Bevorzugt sind hoch oxidierte niedrig sulfitierte natürliche Öle bzw. Fette, insbesondere hoch oxidiertes niedrig sulfitiertes Rapsöl bzw. Fischöl.

Unter hoch oxidiert im Sinne der vorliegenden Erfindung ist zu verstehen, dass $\Delta d$, der Unterschied der Dichten von Öl oder Fett nach und vor der Oxidation, im Bereich von 0,01 bis 0,1, bevorzugt von 0,03 und 0,05 liegt. Unter niedrig sulfitiert im Sinne der vorliegenden Erfindung ist zu verstehen, das man oxidiertes Neutralöl oder oxidiertes Neutralfett mit 2 bis 8 Gew.-%, bevorzugt 3 bis 5 Gew.-% eines oder mehrerer Sulfite, gerechnet als $Na_2S_2O_5$, umsetzt.

Geeignete synthetische hydrophobe Stoffe sind beispielsweise gewählt aus Mineralölen, Weißölen, Silikonölen, synthetischen Wachsen und hydrophoben (Co)Polymeren. Als Beispiele für Silikonöle seien Polydimethylsilikone mit einer dynamischen Viskosität im Bereich von 10 bis 700 mPa·s, bevorzugt im Bereich von 30 bis 500 mPa·s genannt, bestimmt jeweils nach DIN EN ISO 3219 bei 23 ˚C. Weitere geeignete Silikonöle sind beispielsweise Polydimethylsilikone, in denen 1 bis 50 mol-% der Methylgruppen durch Phenylgruppen oder $C_2$-$C_{10}$-Alkylgruppen, ersetzt sind, mit einer dynamischen Viskosität im Bereich von 10 bis 700 mPa·s, bevorzugt im Bereich von 30 bis 500 mPa·s genannt, bestimmt jeweils nach DIN EN ISO 3219 bei 23 ˚C.

Als Beispiele für synthetische Wachse seien Polyethylenwachse oder Ethylencopolymerwachse genannt, wie

sie beispielsweise durch radikalische Polymerisation von Ethylen oder radikalische Copolymerisation von Ethylen mit beispielsweise (Meth)acrylsäure oder durch Ziegler-Natta-Katalyse erhältlich sind. Weiterhin seien Polyisobutylenwachse genannt. Weiterhin seien Paraffingemische genannt; darunter sind Gemische von Kohlenwasserstoffen zu verstehen, die 12 oder mehr Kohlenstoffatome aufweisen und üblicherweise einen Schmelzpunkt im Bereich von 25 bis 45 ˚C aufweisen. Derartige Paraffingemische können beispielsweise in Raffinerien oder Crackern anfallen und sind dem Fachmann als Paraffingatsch und Sasolwachse bekannt. Ein weiteres Beispiel für synthetische Wachse sind Montanesterwachse.

Hydrophobe (Co)Polymere im Rahmen der vorliegenden Erfindung können auch selbstemulgierend sein und sind beispielsweise gewählt aus

- nicht, partiell oder quantitativ hydrolysierte Copolymerisaten von ethylenisch ungesättigten $C_4$-$C_{10}$-Dicarbonsäureanhydriden wie beispielsweise Maleinsäureanhydrid oder Itaconsäureanhydrid und $C_{10}$-$C_{40}$-$\alpha$-Olefinen,
- Polyisobutenen mit einem Molekulargewicht $M_n$ im Bereich von 500 bis 500.000 g/mol, bevorzugt 1000 bis 100.000 g/mol,
- nicht, partiell oder quantitativ hydrolysierte Copolymerisaten von ethylenisch ungesättigten $C_4$-$C_{10}$-Dicarbonsäureanhydriden wie beispielsweise Maleinsäureanhydrid oder Itaconsäureanhydrid und Vinylaromaten wie beispielsweise Styrol oder para-Methylstyrol,
- statistische Copolymerisaten von Vinylaromaten wie beispielsweise Styrol und ethylenisch ungesättigten $C_3$-$C_{10}$-Carbonsäuren wie beispielsweise (Meth)acrylsäure,
- Copolymerisaten von Butadien und Styrol,
- Blockcopolymerisaten von Vinylaromaten wie beispielsweise Styrol und ethylenisch ungesättigten $C_3$-$C_{10}$-Carbonsäuren wie beispielsweise (Meth)acrylsäure,
- Copolymerisaten, insbesondere Blockcopolymerisaten von Isobuten und Alkylenoxid, insbesondere Ethylenoxid,

Hydrophobe Stoffe und insbesondere synthetische hydrophobe Stoffe, die als Komponente (b) geeignet sind, können durchaus selbstemulgierende Eigenschaften aufwei-So sind als hydrophobe Copolymere beispielsweise Copolymere von $\alpha$-$C_{20}$-$C_{24}$-Olefinen und Maleinsäureanydrid in einem beliebigen Molverhältnis, insbesondere im Molverhältnis 1:1 geeignet, weiterhin oxidierte Polyethylenwachse, beispielsweise solche mit einer Säurezahl im Bereich von 1 bis 150 mg KOH/g Wachs, bestimmt nach DIN 53402.

Erfindungsgemäße Zusammensetzungen enthalten weiterhin Wasser (c), und zwar mindestens 20 Gew.-%, bezogen auf das Gesamtgewicht an erfindungsgemäßer Zusammensetzung, bevorzugt mindestens 40 Gew.-%.

Die erfindungsgemäßen Zusammensetzungen sind dadurch gekennzeichnet, dass sie einen mittleren hydrodynamischen Radius im Bereich von 1 bis 200 nm aufweisen, bevorzugt 10 bis 120 nm und besonders bevorzugt 20 bis 75 nm, bestimmt durch dynamische Lichtstreuung. Zur Bestimmung des mittleren hydrodynamischen Radius kann man nach dem Fachmann an sich bekannten Methoden den Eigendiffusionskoeffizienten bestimmen und nach der Stokes-Einstein-Gleichung den mittleren hydrodynamischen Radius errechnen.

Erfindungsgemäße Zusammensetzungen weisen in wässriger Verdünnung von 1: 499 (Volumenanteile) eine Transmission von mindestens 80%, bevorzugt 80 bis 95% und besonders bevorzugt von 85 bis 90% auf, gemessen bei einer Wellenlänge von 400 nm und einer Probenschichtdicke von 1 cm. Die Transmission lässt sich beispielsweise in handelsüblichen UV/Vis-Spektrometern bequem bestimmen.

In einer Ausführungsform der vorliegenden Erfindung können erfindungsgemäße Zusammensetzungen bei 20˚C eine dynamische Viskosität $\eta$ im Bereich von 0,5 bis 20 mPa·s aufweisen, bestimmt beispielsweise mit Hilfe einer dynamischen Zylinder-Messmethode.

Erfindungsgemäße Zusammensetzungen erweisen sich als sehr stabil. Ihre Tendenz zu brechen ist äußerst gering. Auch in Gegenwart von salzhaltigen wässrigen Lösungen und insbesondere gegenüber Calciumionen-haltigem, sogenanntem hartem Wasser mit einer Härte von 21˚ dH und mehr bleiben sie stabil. Auch in Gegenwart von wässrigen Lösungen von Soda, Ameisensäure, Glaubersalz oder Chromsalzen bleiben sie stabil.

In einer Ausführungsform der vorliegenden Erfindung können erfindungsgemäße Zusammensetzungen

(d) mindestens einen ionischen oder nichtionischen Emulgator
enthalten.

Geeignete ionische Emulgatoren sind ausgewählt aus kationischen, anionischen und zwitterionischen Emulgatoren.

Als Emulgatoren (d) können im Prinzip alle in wässrigen Systemen oberflächenaktiven Verbindungen eingesetzt werden, die nichtionischer, anionischer, kationischer oder auch zwitterionischer Natur sein können.

Besonders gut geeignete Emulgatoren (d) sind N-acylierte Aminosäurederivate beispielsweise der Formel I

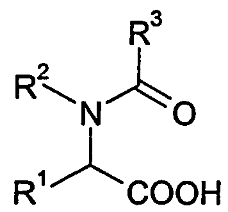

I

in denen die Variablen wie folgt definiert sind:

$R^1$ Wasserstoff, $C_1$-$C_4$-Alkyl, wie Methyl, Ethyl, n-Propyl, iso-Propyl, n-Butyl, iso-Butyl, sec.-Butyl und tert.-Butyl, insbesondere Methyl; $C_6$-$C_{14}$-Aryl, beispielsweise Phenyl, 1-Naphthyl, 2-Naphthyl, 1-Anthryl, 2-Anthryl, 9-Anthryl, 1-Phenanthryl, 2-Phenanthryl, 3-Phenanthryl, 4-Phenanthryl und 9-Phenanthryl, bevorzugt Phenyl, 1-Naphthyl und 2-Naphthyl, besonders be- vorzugt Phenyl;

$R^2$ $C_1$-$C_4$-Alkyl, wie Methyl, Ethyl, n-Propyl, iso-Propyl, n-Butyl, iso-Butyl, sec.-Butyl und tert.-Butyl; insbesondere Methyl;

Die Gruppe CO-$R^3$ ist üblicherweise von gesättigten oder ungesättigten Fettsäuren abgeleitet. Unter gesättigten Fettsäuren sind Carbonsäuren mit $C_9$-$C_{20}$-Alkyl-gruppen zu verstehen, die linear linear oder verzweigt sein können, substituiert oder unsubstituiert. $R^3$ kann beispielsweise sein: n-Nonyl, n-Decyl, n-Dodecyl, n-Tetradecyl, n-Pentadecyl, n-Octadecyl, n-Eicosyl.

CO-$R^3$ kann von einer ungesättigten Fettsäure mit 9 bis 20 C-Atomen und einer bis 5 C-C-Doppelbindungen abgeleitet sein, wobei die C-C-Doppelbindungen beispielsweise isoliert oder allylisch sein können, beispielsweise der Acylrest der Linolsäure, der Linolensäure, und ganz besonders bevorzugt der Ölsäure.

In einer Ausführungsform der vorliegenden Erfindung sind alle oder zumindest ein gewisser Anteil, beispielsweise ein Drittel oder die Hälfte, der Carboxylgruppen in als Emulgatoren eingesetzten N-acylierten Aminosäurederivaten neutralisiert. Zur Neutralisation eignen sich beispielsweise basische Salze wie Hydroxide oder Carbonate der Alkalimetalle wie beispielsweise Na oder K. Zur Neutralisation eignen sich weiterhin Ammoniak, Alkylamine wie beispielsweise Methylamin, Dimethylamin, Trimethylamin, Ethylamin, Diethylamin, Triethylamin, Ethylendiamin, und ganz besonders Alkanolamine wie beispielsweise Ethanolamin, Diethanolamin, Triethanolamin, N-Methyl-Ethanolamin, N-Methyldiethanolamin oder N-(n-Butyl)-diethanolamin. Je nach Neutralisierungsgrad und pH-Wert handelt es sich bei Emulgatoren der allgemeinen Formel I also um kationische, anionische oder zwitterionische Emulgatoren handeln.

Als beispielhafte Vertreter für Verbindungen der Formel I seien N-Oleylsarcosin, N-Stearylsarcosin, N-Lauroylsarcosin und N-Isononanoylarcosin sowie die jeweiligen Ethanolammoniumsalze, Diethanolammoniumsalze sowie N-Methyldiethanolammoniumsalze genannt.

In einer anderen Ausführungsform der vorliegenden Erfindung handelt es sich bei Emulgatoren (d) um sulfitierte Bernsteinsäurehalbester oder sulfitierte Bernsteinsäureester der allgemeinen Formel II

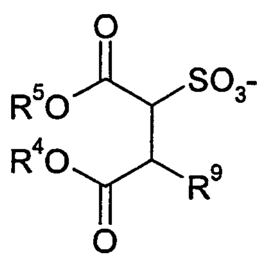

II

$R^4$, $R^5$ gleich oder vorzugsweise verschieden und gewählt aus Wasserstoff,
$C_1$-$C_{30}$-Alkyl, verzweigt oder unverzweigt, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, iso-Heptyl, n-Octyl, n-Nonyl, n-Decyl, n-Undecyl, n-Dodecyl, n-Tridecyl, n-Tetradecyl, n-Hexadecyl, n-Octadecyl, n-Eicosyl, bevorzugt in β-Stellung verzweigte Reste der Formel II a

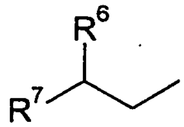

II a

$(CH_2CH_2O)_x$-O-$R^8$ oder $[CH(CH_3)CH_2O)_x$-O-$R^8$, wobei x eine ganze Zahl im Bereich von 1 bis 20 ist, $C_6$-$C_{14}$-Aryl, beispielsweise Phenyl, 1-Naphthyl, 2-Naphthyl, 1-Anthryl, 2-Anthryl, 9-Anthryl, 1-Phenanthryl, 2-Phenanthryl, 3-Phenanthryl, 4-Phenanthryl und 9-Phenanthryl, bevorzugt Phenyl, 1-Naphthyl und 2-Naphthyl, besonders bevorzugt Phenyl;

$R^9$ ist gewählt aus $C_1$-$C_4$-Alkyl wie Methyl, Ethyl, n-Propyl, iso-Propyl, n-Butyl, iso-Butyl, sec.-Butyl, tert.-Butyl Und insbesondere Wasserstoff;

$R^6$, $R^7$ gleich oder vorzugsweise verschieden und gewählt aus $C_1$-$C_{27}$-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, iso-Heptyl, n-Octyl, n-Nonyl, n-Decyl, n-Un- decyl, n-Dodecyl, n-Tridecyl, n-Tetradecyl, n-Hexadecyl, n-Octadecyl, n-Eicosyl; wobei die Summe der C-Atome von $R^6$ und $R^7$ maximal 30 beträgt. Vorzugswei- se hat $R^6$ zwei C-Atome mehr als $R^7$; bevorzugt sind beispielsweise die Kombi- nationen

$R^6$ = n-Undecyl und $R^7$ = n-Nonyl, $R^6$ = n-Dodecyl und $R^7$ = n-Decyl, $R^6$ = n-Tridecyl und $R^7$ = n-Undecyl, $R^6$ = n-Tetradecyl und $R^7$ = n-Dodecyl, $R^6$ = n-Pentadecyl und $R^7$ = n-Tridecyl.

$R^8$ ist gewählt aus $C_1$-$C_4$-Alkyl wie Methyl, Ethyl, n-Propyl, iso-Propyl, n-Butyl, iso-Butyl, sec.-Butyl, tert.-Butyl, Phenyl, ortho-Tolyl, meta-Tolyl, para-Tolyl und insbesondere Wasserstoff.

In einer bevorzugten Ausführungsform der vorliegenden Erfindung ist genau einer der Reste $R^4$ und $R^5$ Wasserstoff und der andere Rest gewählt aus $C_1$-$C_{30}$-Alkyl.

In einer besonders bevorzugten Ausführungsform der vorliegenden Erfindung wählt man ein Gemisch von mehreren Verbindungen beispielsweise der Formel II, die sich beispielsweise dadurch unterscheiden können, dass in der ersten Verbindung der Formel II $R^4$ gleich Wasserstoff und $R^5$ aus $C_1$-$C_{30}$-Alkyl gewählt wird und in der zweiten $R^5$ gleich Wasserstoff und $R^4$ aus $C_1$-$C_{30}$-Alkyl gewählt wird.

In einer Ausführungsform der vorliegenden Erfindung sind alle oder zumindest ein gewisser Anteil, beispielsweise ein Drittel oder die Hälfte, der Sulfonylgruppen in als Emulgator (d) eingesetzten Verbindungen der allgemeinen Formel II neutralisiert. Zur Neutralisation eignen sich beispielsweise basische Salze wie Hydroxide oder Carbonate der Alkalimetalle wie beispielsweise Na oder K. Zur Neutralisation eignen sich weiterhin Ammoniak, Alkylamine wie beispielsweise Methylamin, Dimethylamin, Trimethylamin, Ethylamin, Diethylamin, Triethylamin, Ethylendiamin, und ganz besonders Alkanolamine wie beispielsweise Ethanolamin, Diethanolamin, Triethanolamin, N-MethylEthanolamin, N-Methyldiethanolamin oder N-(n-Butyl)-diethanolamin.

Die Herstellung von Verbindungen der Formel II ist an sich bekannt und in WO 01/68584 beschrieben. Sie gelingt beispielsweise durch einfache oder doppelte Veresterung von Dicarbonsäureanhydriden der allgemeinen Formel III

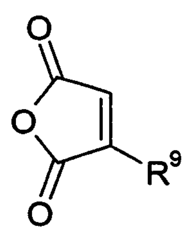

III

mit entsprechenden Alkoholen, die nicht rein vorliegen müssen, gefolgt von einer Umsetzung mit Disulfit.

Man kann anstatt reiner Schwefel-haltiger Verbindungen, beispielsweise Schwefel-haltiger Verbindungen der Formel V, Gemische von verschiedenen Schwefel-haltigen Verbindungen verwenden. Beispielsweise ist es möglich, zur Veresterung das als Oxoöl 135 oder Oxodicköl 135 (WO 01/68584) bekannte Gemisch einzusetzen.

In einer Ausführungsform der vorliegenden Erfindung können im erfindungsgemäßen Verfahren eingesetzte Formulierungen bis zu 40 Gew.-%, bevorzugt bis zu 20 Gew.-%, bezogen auf Formulierung, mindestens eines Alkohols der Formel IV

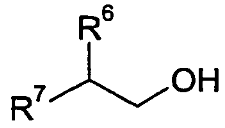

IV

enthalten, wobei in den Formeln III und IV die Variablen wie oben stehend definiert sind.

In einer Ausführungsform der vorliegenden Erfindung können erfindungsgemäße Zusammensetzungen

(e) mindestens einen Zuschlagstoff

enthalten.

An Zuschlagstoffen sind beispielsweise zu nennen: Gerbstoffe, insbesondere polymere Gerbstoffe wie beispielsweise beschrieben in EP-B 0 891 430, vegetabilische Gerbstoffe und synthetische Gerbstoffe, beispielsweise beschrieben in EP-A 0 459 168, und weiterhin Dispergiermittel, beispielsweise beschrieben in EP-A2 0 463 401.

In einer Ausführungsform der vorliegenden Erfindung sind erfindungsgemäße Zusammensetzungen im Wesentlichen frei von niedermolekularen Alkoholen und niedermolekularen Glykolen. Unter niedermolekularen Alkoholen sind im Zusammenhang der vorliegenden Erfindung Alkohole mit bis zu 8 C-Atomen, besonders Alkanale mit bis zu 8 C-Atomen und insbesondere Ethanol, Isopropanol und n-Pentanol zu nennen. Unter niedermolekularen Glykolen im Sinne der vorliegenden Erfindung sind Verbindungen mit bis zu 6 C-Atomen und mindestens zwei alkoholischen Hydroxylgruppen zu verstehen. Beispielhaft seien Ethylenglykol, Propylenglykol, Isopropylenglykol, 1,4-Butandiol, 1,2-Butandiol, 2,3-Butandiole, Isobutenglykol, 1,3-Butandiol, 1,2-Hexandiol und 1,6-Hexandiol genannt, aber auch Glycerin, Trimethylolpropan und isomere 2,3,4-Propantriole. Im Wesentlichen frei bedeutet im Sinne der vorliegenden Erfindung beispielsweise, dass erfindungsgemäße Zusammensetzungen weniger als 0,1 Gew.-%, bevorzugt weniger als 0,01 Gew.-% an niedermolekularen Alkoholen bzw. Glykol enthalten und insbesondere im Bereich von 10 ppm bis 0,05 Gew.-% an niedermolekularen Alkoholen und Glykol zusammen, wobei Angaben in Gew.-% jeweils auf das Gesamtgewicht an erfindungsgemäßer Zusammensetzung bezogen sind.

In einer Ausführungsform der vorliegenden Erfindung haben erfindungsgemäße Zusammensetzungen einen pH-Wert im Bereich von 3 bis 7, bevorzugt im Bereich von 4 bis 6.

In einer Ausführungsform der vorliegenden Erfindung enthalten erfindungsgemäße Zusammensetzungen
0,1 bis 50 Gew.-%, bevorzugt 2,5 bis 10 Gew.-% an Komponente (a),
5 bis 50 Gew.-%, bevorzugt 10 bis 40 Gew.-% an Komponente (b),
20 bis 90 Gew.-%, bevorzugt 40 bis 80 Gew.-% an Wasser (c),
0 bis 40 Gew.-%, bevorzugt 1 bis 20 Gew.-% an ionischem oder nichtionischen Emulgator (d),
0 bis 50 Gew.-%, bevorzugt 15 bis 30 Gew.-% an Zuschlagstoffen (e),
wobei Angaben in Gew.-% jeweils auf das Gesamtgewicht der betreffenden erfindungsgemäßen Zusammensetzung bezogen ist.

Ein weiterer Gegenstand der vorliegenden Erfindung ist ein Verfahren zur Herstellung erfindungsgemäßer Zusammensetzungen, im Folgenden auch als erfindungsgemäßes Herstellverfahren bezeichnet.

In einer Ausführungsform des erfindungsgemäßen Herstellungsverfahrens geht man so vor, dass man

(a) mindestens eine Mischung von mindestens zwei verschiedenen alkoxylierten Alkoholen,
(b) mindestens eine hydrophobe Komponente, gewählt aus natürlichen, modifizierten natürlichen und synthetischen hydrophoben Stoffen,
(d) gegebenenfalls mindestens einen ionischen oder nichtionischen Emulgator und
(e) gegebenenfalls mindestens einen Zuschlagstoff
miteinander mischt und anschließend
(c) mindestens 20 Gew.-%, bevorzugt mindestens 40 Gew.-% Wasser zugibt, bezogen auf das Gesamtgewicht der Zusammensetzung,

wobei das Wasser (c) bei der Zugabe eine Temperatur von mindestens 70˚C aufweist, bevorzugt im Bereich von 80˚C bis 100˚C.

Vorzugsweise gibt man Wasser (c) langsam zu. Langsam heißt im Zusammenhang mit der vorliegenden Erfindung, dass man nicht mehr als 1 Liter Wasser (c), das bei der Zugabe eine Temperatur von mindestens 70˚C

aufweist, über den Zeitraum von 5 Minuten zugibt. Üblicherweise vermeidet man es, Wasser (c), das bei der Zugabe eine Temperatur von mindestens 70˚C aufweist, in einer Portion und innerhalb von 3 Minuten pro Liter oder weniger zuzugeben.

Nach der Zugabe von Wasser (c), das bei der Zugabe eine Temperatur von mindestens 70˚C aufweist, bevorzugt im Bereich von 80˚C bis 100˚C, kann man beispielsweise unter Durchmischen auf Zimmertemperatur abkühlen lassen. Man kann auch nach Zugabe von Wasser (c), das eine Temperatur von mindestens 70˚C aufweist, bevorzugt im Bereich von 80˚C bis 100˚C, über einen Zeitraum von 5 Minuten bis 10 Stunden bei einer Temperatur oberhalb Zimmertemperatur, beispielsweise bei 70˚C oder insbesondere im Bereich von 80 bis 100˚C, nachrühren und erst danach auf Zimmertemperatur abkühlen.

In einer anderen Ausführungsform des erfindungsgemäßen Herstellungsverfahrens geht man beispielsweise so vor, dass man

(a) mindestens eine Mischung von mindestens zwei verschiedenen alkoxylierten Alkoholen,
(b) mindestens eine hydrophobe Komponente, gewählt aus natürlichen, modifizierten natürlichen und synthetischen hydrophoben Stoffen,
(c) mindestens 20 Gew.-%, bevorzugt mindestens 40 Gew.-% Wasser, bezogen auf das Gesamtgewicht an erfindungsgemäßer Zusammensetzung,
(d) gegebenenfalls mindestens einen ionischen oder nichtionischen Emulgator und
(e) gegebenenfalls mindestens einen Zuschlagstoff
miteinander mischt, beispielsweise bei Zimmertemperatur oder einer Temperatur im Bereich von 20 bis 50˚C, und anschließend die so erhältliche Mischung auf eine Temperatur von mindestens 70˚C, bevorzugt auf eine Temperatur im Bereich von mindestens 80˚C bis 100˚C, erwärmt.

Bei dieser Ausführungsform des erfindungsgemäßen Herstellverfahrens kann man zum Mischen Wasser (c) einsetzen, das eine beliebige Temperatur beispielsweise im Bereich von 0 bis 100˚C aufweist. Aus praktischen Gründen kann man zum Mischen Wasser (c) einsetzen, welches eine Temperatur im Bereich von 10 bis 30˚C aufweist.

Im Anschluss an das Erwärmen auf eine Temperatur von mindestens 70˚C, bevorzugt auf eine Temperatur im Bereich von mindestens 80˚C bis 100˚C, kann man, gegebenenfalls unter weiterem Rühren, die Mischung über einen Zeitraum von mehreren Minuten bis 24 Stunden, bevorzugt mindestens eine Stunde bis 8 Stunden, bei einer Temperatur von mindestens 70˚C halten und erst danach wieder auf Zimmertemperatur abkühlen.

In einer anderen Ausführungsform des erfindungsgemäßen Herstellungsverfahrens geht man beispielsweise so vor, dass man

(a) mindestens eine Mischung von mindestens zwei verschiedenen alkoxylierten Alkoholen,
(b) mindestens eine hydrophobe Komponente, gewählt aus natürlichen, modifizierten natürlichen und synthetischen hydrophoben Stoffen,
(c) mindestens 20 Gew.-%, bevorzugt mindestens 40 Gew.-% Wasser, bezogen auf das Gesamtgewicht an erfindungsgemäßer Zusammensetzung, das
(d) gegebenenfalls mindestens einen ionischen oder nichtionischen Emulgator
(e) gegebenenfalls mindestens einen Zuschlagstoff enthält,

miteinander mischt, beispielsweise bei Zimmertemperatur oder einer Temperatur im Bereich von 20 bis 50˚C, und anschließend die so erhältliche Mischung auf eine Temperatur von mindestens 70˚C, bevorzugt auf eine Temperatur im Bereich von mindestens 80˚C bis 100˚C, erwärmt.

Bei dieser Ausführungsform des erfindungsgemäßen Herstellverfahrens kann man zum Mischen gegebenenfalls mindestens einen ionischen oder nichtionischen Emulgator (d) und gegebenenfalls mindestens einen Zuschlagstoff enthaltendes Wasser (c) einsetzen, das eine beliebige Temperatur beispielsweise im Bereich von 0 bis 100˚C aufweist. Aus praktischen Gründen kann man zum Mischen Wasser (c) einsetzen, welches eine Temperatur im Bereich von 10 bis 30˚C aufweist.

Im Anschluss an das Erwärmen auf eine Temperatur von mindestens 70˚C, bevorzugt auf eine Temperatur im Bereich von mindestens 80˚C bis 100˚C, kann man, gegebenenfalls unter weiterem Rühren, die Mischung über einen Zeitraum von mehreren Minuten bis 24 Stunden, bevorzugt mindestens eine Stunde bis 8 Stunden, bei einer Temperatur von mindestens 70˚C halten und erst danach wieder auf Zimmertemperatur abkühlen.

Ein weiterer Gegenstand der vorliegenden Erfindung ist die Verwendung von erfindungsgemäßen Zusammensetzungen bzw. von Zusammensetzungen, die nach dem erfindungsgemäßen Herstellverfahren hergestellt sind, zur Herstellung von Leder oder Pelzfellen. Ein weiterer Gegenstand der vorliegenden Erfindung ist ein Verfahren zur Herstellung von Leder oder Pelzfellen unter Verwendung von erfindungsgemäßen Zusammensetzungen bzw. von nach dem erfindungsgemäßen Herstellverfahren hergestellten Zusammensetzungen, im Folgenden auch erfindungsgemäßes

Fettungsverfahren genannt.

Zur Durchführung des erfindungsgemäßen Fettungsverfahren behandelt man Leder oder Pelzfelle in einer üblicherweise wässrigen Flotte vor, während oder nach der Nachgerbung mit mindestens einer erfindungsgemäß eingesetzten Zusammensetzung. Das erfindungsgemäße Fettungsverfahren kann man einmal oder wiederholt durchführen. Die zu fettenden Leder können nach beliebigen Methoden hergestellt worden sein, beispielsweise durch Mineralgerbung, insbesondere Chromgerbung, oder durch Polymergerbung, Gerbung mit Syntanen, Harzgerbung, Gerbung mit vegetabilen Gerbstoffen oder auch Gerbung mit Kombinationen aus den vorgenannten Gerbstoffen.

In einer Ausführungsform des erfindungsgemäßen Fettungsverfahrens wird mindestens eine erfindungsgemäße Zusammensetzung in einer oder mehreren Portionen zu dem zu fettenden Leder oder den zu fettenden Pelzen gegeben. Vorzugsweise kann die Flottenlänge 50 bis 2000 Gew.-%, bevorzugt 100 bis 400 Gew.-% betragen, bezogen auf das Falzgewicht der Leder bzw. das Nassgewicht der Pelzfelle.

Das erfindungsgemäße Fettungsverfahren führt man im Allgemeinen so durch, dass man das zu fettende Leder bzw. die zu fettenden Pelzfelle in geeigneten Gefäßen, beispielsweise in Fässern, insbesondere in drehbaren Fässern mit Einbauten, walkt. Auch andere dem Fachmann bekannte Methoden zur Durchmischung sind möglich.

Als Temperatur für das erfindungsgemäße Verfahren kann man Temperaturen im Bereich von 20 bis 65°C, bevorzugt 30 bis 60° wählen.

Die Druckbedingungen des erfindungsgemäßen Verfahrens sind im Allgemeinen unkritisch. Bevorzugt arbeitet man bei Normaldruck (1 atm), man kann aber auch bei verringertem Druck wie beispielsweise 0,5 bis 0,99 atm oder bei erhöhtem Druck wie beispielsweise 1,01 bis 2 atm arbeiten.

Als pH-Wert kann man zu Beginn des erfindungsgemäßen Fettungsverfahrens einen pH-Wert im Bereich von 4 bis 8, bevorzugt 4,5 bis 8 einstellen. Am Ende des erfindungsgemäßen Fettungsverfahrens kann man den pH-Wert durch Zugabe einer Säure, beispielsweise Ameisensäure, auf einen pH-Wert von 3 bis 5 absenken.

Das erfindungsgemäße Fettungsverfahren ist im Allgemeinen nach einer Zeit von 20 Minuten bis 24 Stunden beendet, bevorzugt 30 Minuten bis 12 Stunden. Wenn man das erfindungsgemäße Fettungsverfahren wiederholt durchführt, so spricht man im Rahmen der vorliegenden Erfindung von erfindungsgemäßen Fettungsschritten.

Man kann beispielsweise 0,1 bis 20 Gew.-%, insbesondere 0,5 bis 15 Gew.-% erfindungsgemäße Zusammensetzung pro erfindungsgemäßem Fettungsschritt bzw. im erfindungsgemäßen Fettungsverfahren einsetzen, bezogen auf das Falzgewicht der zu behandelnden Leder bzw. das Nassgewicht der zu behandelnden Pelze.

Während des erfindungsgemäßen Fettungsverfahrens kann man der Flotte übliche Lederfarbstoffe zusetzen. Beispielhaft seien saure, substantive oder basische Anilinfarbstoffe in Betracht, die in gerbereiüblichen Mengen eingesetzt werden können.

Wünscht man das erfindungsgemäße Fettungsverfahren in Kombination mit der Nachgerbung durchzuführen, so kann man mit beliebigen in der Gerberei üblichen Gerbstoffen, beispielsweise Mineralgerbstoffen, insbesondere Chromgerbstoffen, oder Polymergerbstoffen, Syntanen, Harzgerbstoffen, vegetabilen Gerbstoffen oder Kombinationen aus den vorgenannten Gerbstoffen arbeiten, die man getrennt oder zusammen mit erfindungsgemäßer Zusammensetzung zugeben kann.

Man beobachtet, dass die Auszehrung der erfindungsgemäßen Zusammensetzungen ausgezeichnet sind; Restflotten (Abwässer), die nach Ausübung des erfindungsgemäβen Fettungsverfahrens verbleiben und üblicherweise entsorgt werden, haben einen reduzierten Chemischen Sauerstoffbedarf.

Im Anschluss an das erfindungsgemäße Fettungsverfahren kann man die erfindungsgemäß gefetteten Leder bzw. Pelzfelle wie gerbereitechnisch üblich aufarbeiten.

Nach dem erfindungsgemäßen Fettungsverfahren erhaltene Leder und Pelzfelle, im Folgenden auch erfindungsgemäß gefettete Leder und Pelzfelle genannt, zeichnen sich durch besonders gute Griffeigenschaften, sehr niedrige Fogging-Werte und angenehme mechanische Eigenschaften aus.

Ein weiterer Gegenstand der vorliegenden Erfindung ist die Verwendung erfindungsgemäß gefetteter Leder zur Herstellung von Bekleidungsstücken, beispielsweise Jacken, Mänteln, Schuhen und insbesondere Stiefeln. Ein weiterer Gegenstand der vorliegenden Erfindung ist die Verwendung erfindungsgemäß gefetteter Leder zur Herstellung von Möbeln und Möbelteilen, beispielsweise Ledersofas, Ledersesseln, Armlehnen für Stühle, Sessel oder Sofas oder Bänke. Ein weiterer Gegenstand der vorliegenden Erfindung ist die Verwendung erfindungsgemäß gefetteter Leder zur Herstellung von Autoteilen, beispielsweise Autositzen, Teilen von Armaturenbrettern und Innenverkleidungsteilen, beispielsweise in Autotüren.

Die Erfindung wird durch Arbeitsbeispiele erläutert.

Allgemeine Bemerkungen:

Die Bestimmungen des mittleren hydrodynamischen Radius erfolgten mit einem Laser-Goniometer der Firma ALV-Laser GmbH, Langen (Model ALV/SP-86). Als Lichtquelle fungierte ein He-Ne-Laser (Model LHRP-3501 von Laser2000) mit einer Wellenlänge von 633 nm und einer Leistung von 35 mW. Zur Detektion wurde eine ALV/High

Quantum Efficiency Avalanche Photodiode/ High Performance AQ-Detektor mit vorgeschaltetem ALVI Static and Dynamic Enhancer eingesetzt. Ein Thermostat (MWG Lauda Model RK 20) hielt die Probentemperatur bei 23°C.

Bei der Größenbestimmung mittels Photonenkorrelationsspektroskopie erhielt man den hydrodynamischen Radius

$$r_H = \frac{kT}{6\pi\eta\langle D\rangle_z},$$

wo $kT$ für die thermische Energie, η für die dynamische Viskosität und

$$\langle D\rangle_z = \frac{\int D(r)m^2(r)dr}{\int m^2(r)dr}$$

(m(r): Masse eines Teilchens vom Radius r) das Z-Mittel des Diffusionskoeffizienten $D(r) = \frac{kT}{6\pi\eta\ r}$ steht.

Die Breite der Verteilung wurde durch den "Coefficient of Variation" (CV) angegeben; dies ist die mit dem Mittelwert normierte Standardabweichung bezüglich der Intensitäts-oder Z-Mittelung:

$$CV = \left[\frac{\langle D^2\rangle_z - \langle D\rangle_z^2}{\langle D\rangle_z^2}\right]^{1/2}.$$

(I) Herstellung von erfindungsgemäßen Zusammensetzungen und Vergleichs zusammensetzungen

I.1. Herstellung von erfindungsgemäßer Zusammensetzung Z1

In einem 1-Liter-Rührgefäß mit Heizvorrichtung wurden miteinander vermischt:

| | |
|---|---|
| 7,50 g | $n\text{-}C_{10}H_{21}\text{-}O\text{-}(CH_2\text{-}CH_2\text{-}O)_3\text{-}H$ |
| 7,50 g | $n\text{-}C_{10}H_{21}\text{-}O\text{-}(CH_2\text{-}CH_2\text{-}O)_4\text{-}H$ |

250 g Rapsölmethylester (B1) mit einer dynamischen Viskosität von 4,5 mPa·s, gemessen nach DIN EN ISO 3219 bei 23 °C.

Die so erhaltene Mischung wurde auf 85°C erwärmt. Anschließend wurden über einen Zeitraum von 15 Minuten 250 g destilliertes Wasser, das eine Temperatur von 80°C aufwies, tropfenweise zugegeben und während der Zugabe kräftig (20.000 Umdrehungen/min) gerührt. Man rührte weitere 30 Minuten kräftig nach und kühlte anschließend in einem Eisbad auf Zimmertemperatur ab. Man erhielt erfindungsgemäße Zusammensetzung Z1.

I.2. Herstellung von erfindungsgemäßer Zusammensetzung Z2

In einem 1-Liter-Rührgefäß mit Heizvorrichtung wurden miteinander vermischt:

| | |
|---|---|
| 2,30 g | $n\text{-}C_{18}H_{37}\text{-}O\text{-}(CH_2\text{-}CH_2\text{-}O)_{25}\text{-}H$ |
| 2,08 g | $n\text{-}C_{10}H_{21}\text{-}O\text{-}(CH_2\text{-}CH_2\text{-}O)_7\text{-}H$ |
| 1,62 g | $n\text{-}C_{12}H_{25}\text{-}O\text{-}(CH_2\text{-}CHrO)_{80}\text{-}H$ |
| 240 g | einer hydrophoben Komponente B2, die wie folgt hergestellt wurde: |

Ein Gemisch aus Kapellanöl und Rapsöl (Gew.-Verhältnis 40 : 60) wurde zunächst mit Luft oxidiert, bis ein Dichteunterschied Δd von 0,1 erreicht war, und danach mit 8 Gew.-% $Na_2S_2O_5$ umgesetzt, bezogen auf die Summe an nicht-oxidiertem Gemisch aus Kapellanöl und Rapsöl.

Die so erhaltene Mischung wurde auf 85°C erwärmt. Anschließend wurden über einen Zeitraum von 5 Stunden 360 g destilliertes Wasser, das eine Temperatur von 80°C aufwies, tropfenweise zugegeben und während der Zugabe gerührt. Man rührte weitere 30 Minuten nach und kühlte anschließend in einem Eisbad auf Zimmertemperatur ab. Man erhielt erfindungsgemäße Zusammensetzung Z2.

I.3. Herstellung von Vergleichszusammensetzung V3

7 g n-$C_{18}H_{37}$-O-($CH_2$-$CH_2$-O)$_{25}$-H

240 g einer hydrophoben Komponente B3, die wie folgt hergestellt wurde:

Ein Gemisch aus Kapellanöl und Rapsöl (Gew.-Verhältnis 40 : 60) wurde zunächst mit Luft oxidiert, bis ein Dichteunterschied Δd von 0,1 erreicht war, anschließend mit 9 Gew.-% (bezogen auf die Summe der Massen von Kapellanöl und Rapsöl) einer Mischung aus Kokosfettmonoethanolamin und Maleinsäureanhydrid versetzt (Gew.-Verhältnis 70: 30) und danach mit 12 Gew.-% $Na_2S_2O_5$ umgesetzt, bezogen auf die Summe an Einsatzstoffen.

Die so erhaltene Mischung wurde anschließend mit 360 g destilliertem Wasser, das eine Temperatur von 20°C hatte, unter Rühren verdünnt. Man erhielt Vergleichszusammensetzung V3.

II. Bestimmung von Transmission und mittlerem hydrodynamischem Radius

Von Z1, Z2 und V3 wurde jeweils der mittlere hydrodynamische Radius und die Transmission bestimmt.

Die Transmission wurde jeweils bei 400 nm und einer wässrigen Verdünnung von 1:499 (Volumenanteile) bei einer Probenschichtdicke von 1 cm gemessen. Die Ergebnisse sind aus Tabelle 1 ersichtlich.

Tabelle 1: Eigenschaften von Z1, Z2 und V3

| Probe | Mittlerer hydrodynamischer Radius [nm] | Transmission [%] | pH-Wert |
|---|---|---|---|
| Z1 | 20 | n.b. | 4,8 |
| Z2 | 61 | 90 | 5,1 |
| V3 | 220 | 75 | 5,6 |
| n.b.: nicht bestimmt | | | |

III. Anwendungsbeispiele

III.1. Herstellung von Automobilleder

Die Angaben in Gew.-% beziehen sich jeweils auf das Falzgewicht, wenn nicht anders angegeben. Bei allen Operationen wurde das Fass etwa 10 mal pro Minute gedreht, wenn nicht anders angegeben.

In einem drehbaren 50-l-Fass mit Einbauten wurden 2,5 kg Chrom-gegerbtes Rindsleder (wet blue) mit einer Falzstärke von 1,1 bis 1,3 mm mit 100 Gew.-% Wasser mit einer Temperatur von 35°C, 2 Gew.-% Dispergiermittel 3 aus EP 0 463 401 A2 und 1,6 Gew.-% Natriumformiat und nach einer halben Stunde mit 0,3 Gew.-% $NaHCO_3$ versetzt. Nach 15 Minuten wurden 0,6 Gew.-% $NaHCO_3$ zugegeben und bei 35°C über eine Zeitdauer von 60 Minuten entsäuert, so dass sich ein pH-Wert von 4,9 einstellte. Anschließend wurde das Leder mit Wasser gewaschen, mit 80 Gew.-% Wasser und 2 Gew.-% Dispergiermittel 3 aus EP 0 463 401 A2 versetzt. Man walkte 20 Minuten; es stellte sich ein pH-Wert von 5,8 ein.

Danach gab man 0,6 Gew.-% Polymerisat mit den folgenden charakteristischen Daten zu:

30 Gew.-%ige wässrige, mit NaOH teilneutralisierte Polymerlösung; Homopolymer der Methacrylsäure, $M_n$ ca. 10.000; K-Wert nach Fikentscher: 12, Viskosität der 30 Gew.-% Lösung: 65 mPa·s (DIN EN ISO 3219, 23°C), pH-Wert 5,1. Es wurde 30 Minuten nachgegerbt.

Danach gab man 2 Gew.-% Sulfongerbstoff aus EP-B 0 459 168, Beispiel K1 und 5 Gew.-% Vegetabilgerbstoffs Mimosaextrakt, kommerziell erhältlich bei BASF Aktiengesellschaft, zugesetzt. Die Behandlung wurde über einen Zeitraum von einer Stunde fortgesetzt.

Danach gab man 1 Gew.-% eines schwarzen Farbstoffgemischs zu, das wie folgt zusammengesetzt war:

70 Gewichtsteile schwarzen Farbstoff nach WO 98/41581, Beispiel 2.2 und
30 Gewichtsteile Acid Black 194 (Chromkomplex), Colour Index 194.

Man gab 80 Gew.-% Wasser zu, heizte auf 55˚C auf und setzte erfindungsgemäße Zusammensetzung gemäß Tabelle 2 oder Vergleichszusammensetzung zu. Nach 60 Minuten stellte man mit Ameisensäure einen pH-Wert von 3,7 ein.

Schließlich ließ man die Flotte ab und bestimmte den CSB-Wert. Das Leder wurde zweimal mit Wasser gewaschen, getrocknet und gerbereiüblich aufgearbeitet. Man erhielt die erfindungsgemäßen Leder 3.1 bis 3.2 bzw. das Vergleichsleder V3.3 gemäß Tabelle 2.

Die anwendungstechnischen Eigenschaften der erhaltenen Leder gehen aus Tabelle 2 hervor.

Tabelle 2: Anwendungstechnische Eigenschaften von Leder 3.1 bis 3.2 (erfindungsgemäß) und Vergleichsleder V3.3

| Leder | Eingesetzte Zusammensetzung | Fülle | Weichheit | CSB-Wert Abwasser [mg $O_2$/l] |
|---|---|---|---|---|
| 3.1 | Z1 | sehr gut | gut | 6.690 |
| 3.2 | Z2 | sehr gut | sehr gut | 8.900 |
| V3.3 | V3 | gut | gut | 24.000 |

## Patentansprüche

1. Zusammensetzungen, enthaltend als Komponenten

   (a) mindestens zwei verschiedene alkoxylierte Alkohole,
   (b) mindestens eine hydrophobe Komponente, gewählt aus natürlichen, modifizierten natürlichen und synthetischen hydrophoben Stoffen,
   (c) mindestens 20 Gew.-%, bezogen auf das Gesamtgewicht der Zusammensetzung, an Wasser,
   **dadurch gekennzeichnet, dass** die Zusammensetzungen einen mittleren hydrodynamischen Radius im Bereich von 1 bis 200 nm aufweisen, bestimmt durch dynamische Lichtstreuung,
   und dass die Zusammensetzungen in wässriger Verdünnung von 1 : 499 (Volumenanteile) eine Transmission von mindestens 80% bei einer Wellenlänge von 400 nm und einer Probenschichtdicke von 1 cm aufweisen.

2. Zusammensetzungen nach Anspruch 1, **dadurch gekennzeichnet, dass** sie als Komponente (a) eine Mischung von mindestens drei verschiedenen alkoxylierten Alkoholen enthalten.

3. Zusammensetzungen nach einem der Ansprüche 1 bis 2, **dadurch gekennzeichnet, dass** alkoxylierte Alkohole (a) gewählt sind aus

   (a1) $C_6$-$C_{14}$-Alkanoten, alkoxyliert mit 4 bis 12 Äquivalenten Alkylenoxid,
   (a2) einem oder mehreren $C_{12}$-$C_{24}$-Alkanolen, alkoxyliert mit 15 bis 20 Äquivalenten Alkylenoxid,
   (a3) einem oder mehreren $C_{12}$-$C_{24}$-Alkanolen, alkoxyliert mit 50 bis 100 Äquivalenten Alkylenoxid.

4. Zusammensetzung nach einem der Ansprüche 1 bis 3, **dadurch gekennzeichnet, dass** mindestens eine hydrophobe Komponente gewählt wird aus nativen Fetten und nativen Ölen, hoch oxidiertem niedrig sulfitiertem natürlichen Fetten bzw. Ölen, Mineralöten, Weißölen, Silikonölen, synthetischen Wachsen und hydrophoben (Co)Polymeren.

5. Zusammensetzungen nach einem der Ansprüche 1 bis 4, **dadurch gekennzeichnet, dass** sie

   (c) mindestens 40 Gew.-%, bezogen auf das Gesamtgewicht der Zusammensetzung,
   an Wasser enthalten.

**6.** Zusammensetzungen nach einem der Ansprüche 1 bis 5, **dadurch gekennzeichnet, dass** sie

(d) mindestens einen ionischen oder nichtionischen Emulgator enthalten.

**7.** Zusammensetzungen nach einem der Ansprüche 1 bis 6, **dadurch gekennzeichnet, dass** sie

(e) mindestens einen Zuschlagstoff
enthalten.

**8.** Zusammensetzungen nach einem der Ansprüche 1 bis 7, **dadurch gekennzeichnet, dass** sie im Wesentlichen frei sind von niedermolekularen Alkoholen und niedermolekularen Glykolen.

**9.** Verfahren zur Herstellung von Zusammensetzungen nach einem der Ansprüche 1 bis 8, **dadurch gekennzeichnet, dass** man

(a) mindestens zwei verschiedene alkoxylierte Alkohole,
(b) mindestens eine hydrophobe Komponente, gewählt aus natürlichen, modifizierten natürlichen und synthetischen hydrophoben Stoffen,
(d) gegebenenfalls mindestens einen ionischen oder nichtionischen Emulgator und
(e) gegebenenfalls mindestens einen Zuschlagstoff
miteinander mischt und anschließend
(c) mindestens 20 Gew.-% Wasser, bezogen auf das Gesamtgewicht der Zusammensetzung,
zugibt, wobei das Wasser bei der Zugabe eine Temperatur von mindestens 70˚C aufweist.

**10.** Verfahren nach Anspruch 9, **dadurch gekennzeichnet, dass** das Wasser bei der Zugabe eine Temperatur im Bereich von 80˚C bis 100˚C aufweist.

**11.** Verfahren zur Herstellung von Zusammensetzungen nach einem der Ansprüche 1 bis 8, **dadurch gekennzeichnet, dass** man

(a) mindestens zwei verschiedene alkoxylierte Alkohole,
zierten natürlichen und synthetischen hydrophoben Stoffen,
(c) mindestens 20 Gew.-% Wasser, bezogen auf das Gesamtgewicht der Zusammensetzung,
(d) gegebenenfalls mindestens einen ionischen oder nichtionischen Emulgator und
(e) gegebenenfalls mindestens einen Zuschlagstoff
miteinander mischt und anschließend auf eine Temperatur von mindestens 70˚C erwärmt.

**12.** Verfahren nach einem der Ansprüche 9 bis 11, **dadurch gekennzeichnet, dass** man als Komponente (a) ein Gemisch von mindestens 3 alkoxylierten Alkoholen einsetzt.

**13.** Verwendung von Zusammensetzungen nach einem der Ansprüche 1 bis 8 oder von Zusammensetzungen, hergestellt nach einem Verfahren gemäß einem der Ansprüche 9 bis 12, zur Herstellung von Leder oder Pelzfellen.

**14.** Verfahren zur Herstellung von Leder oder Pelzfellen unter Verwendung von Zusammensetzungen nach einem der Ansprüche 1 bis 8 oder Zusammensetzungen, hergestellt nach einem Verfahren gemäß einem der Ansprüche 9 bis 12.

**Claims**

**1.** A composition comprising, as components,

(a) at least two different alkoxylated alcohols,
(b) at least one hydrophobic component selected from natural, modified natural and synthetic hydrophobic substances,
(c) at least 20% by weight, based on the total weight of the composition, of water,
wherein the composition has a mean hydrodynamic radius in the range from 1 to 200 nm, determined by dynamic light scattering,

and wherein the composition, in an aqueous dilution of 1 : 499 (parts by volume), has a transmittance of at least 80% at a wavelength of 400 nm and a sample layer thickness of 1 cm.

**2.** The composition according to claim 1, which comprises, as component (a), a mixture of at least three different alkoxylated alcohols.

**3.** The composition according to either of claims 1 and 2, wherein alkoxylated alcohols (a) are selected from

(a1) $C_6$-$C_{14}$-alkanols, alkoxylated with from 4 to 12 equivalents of alkylene oxide,
(a2) one or more $C_{12}$-$C_{24}$-alkanols, alkoxylated with from 15 to 20 equivalents of alkylene oxide,
(a3) one or more $C_{12}$-$C_{24}$-alkanols, alkoxylated with from 50 to 100 equivalents of alkylene oxide.

**4.** The composition according to any of claims 1 to 3, wherein at least one hydrophobic component is selected from natural fats and natural oils, highly oxidized low-sulfited natural fats or oils, mineral oils, white oils, silicone oils, synthetic waxes and hydrophobic (co)polymers.

**5.** The composition according to any of claims 1 to 4, which comprises

(c)at least 40% by weight, based on the total weight of the composition, of water.

**6.** The composition according to any of claims 1 to 5, which comprises

(d) at least one ionic or nonionic emulsifier.

**7.** The composition according to any of claims 1 to 6, which comprises

(e) at least one additive.

**8.** The composition according to any of claims 1 to 7, which is substantially free of low molecular weight alcohols and low molecular weight glycols.

**9.** A process for the preparation of compositions according to any of claims 1 to 8, wherein

(a) at least two different alkoxylated alcohols,
(b) at least one hydrophobic component selected from natural, modified natural and synthetic hydrophobic substances,
(d) if desired, at least one ionic or nonionic emulsifier and
(e) if desired, at least one additive are mixed with one another and then (c) at least 20% by weight, based on the total weight of the composition, of water,
are added, water having a temperature of at least 70˚C during the addition.

**10.** The process according to claim 9, wherein the water has a temperature in the range from 80˚C to 100˚C during the addition.

**11.** A process for the preparation of compositions according to any of claims 1 to 8, wherein

(a) at least two different alkoxylated alcohols,
(b) at least one hydrophobic component selected from natural, modified natural and synthetic hydrophobic substances,
(c) at least 20% by weight, based on the total weight of the composition, of water,
(d) if desired, at least one ionic or nonionic emulsifier and
(e) if desired, at least one additive
are mixed with one another and then heated to a temperature of at least 70˚C.

**12.** The process according to any of claims 9 to 11, wherein a mixture of at least 3 alkoxylated alcohols is used as component (a).

**13.** The use of a composition according to any of claims 1 to 8 or of a composition prepared by a process according to

any of claims 9 to 12 for the production of leather or fur skins.

**14.** A process for the production of leather or fur skins using compositions according to any of claims 1 to 8 or compositions prepared by a process according to any of claims 9 to 12.

**Revendications**

**1.** Compositions, contenant en tant que composants

(a) au moins deux alcools alcoxylés différents,
(b) au moins un composant hydrophobe, choisi parmi les substances hydrophobes naturelles, naturelles modifiées et synthétiques,
(c) au moins 20 % en poids, par rapport au poids total de la composition, d'eau,
**caractérisées en ce que** les compositions présentent un rayon hydrodynamique moyen dans la plage allant de 1 à 200 nm, déterminé par diffusion dynamique de lumière,
et **en ce que** les compositions en une dilution aqueuse de 1:499 (parties volumiques) présentent une transmission d'au moins 80 % à une longueur d'onde de 400 nm et une épaisseur de couche d'échantillon de 1 cm.

**2.** Compositions selon la revendication 1, **caractérisées en ce qu'**elles contiennent en tant que composant (a) un mélange d'au moins trois alcools alcoxylés différents.

**3.** Compositions selon l'une quelconque des revendications 1 à 2, **caractérisées en ce que** les alcools alcoxylés (a) sont choisis parmi

(a1) les alcanols en $C_6$-$C_{14}$, alcoxylés avec 4 à 12 équivalents d'oxyde d'alkylène,
(a2) un ou plusieurs alcanols en $C_{12}$-$C_{24}$, alcoxylés avec 15 à 20 équivalents d'oxyde d'alkylène,
(a3) un ou plusieurs alcanols en $C_{12}$-$C_{24}$, alcoxylés avec 50 à 100 équivalents d'oxyde d'alkylène.

**4.** Composition selon l'une quelconque des revendications 1 à 3, **caractérisée en ce qu'**au moins un composant hydrophobe est choisi parmi les graisses naturelles et les huiles naturelles, les graisses ou huiles naturelles hautement oxydées et faiblement sulfitées, les huiles minérales, les huiles blanches, les huiles de silicone, les cires synthétiques et les (co)polymères hydrophobes.

**5.** Compositions selon l'une quelconque des revendications 1 à 4, **caractérisées en ce qu'**elles contiennent

(c) au moins 40 % en poids, par rapport au poids total de la composition, d'eau.

**6.** Compositions selon l'une quelconque des revendications 1 à 5, **caractérisées en ce qu'**elles contiennent

(d) au moins un émulsifiant ionique ou non ionique.

**7.** Compositions selon l'une quelconque des revendications 1 à 6, **caractérisées en ce qu'**elles contiennent

(e) au moins un additif.

**8.** Compositions selon l'une quelconque des revendications 1 à 7, **caractérisées en ce qu'**elles sont essentiellement exemptes d'alcools inférieurs et de glycols inférieurs.

**9.** Procédé de fabrication de compositions selon l'une quelconque des revendications 1 à 8, **caractérisé en ce que**

(a) au moins deux alcools alcoxylés différents,
(b) au moins un composant hydrophobe, choisi parmi les substances hydrophobes naturelles, naturelles modifiées et synthétiques,
(d) éventuellement au moins un émulsifiant ionique ou non ionique et
(e) éventuellement au moins un additif
sont mélangés, puis
(c) au moins 20 % en poids d'eau, par rapport au poids total de la composition,

sont ajoutés, l'eau présentant lors de l'ajout une température d'au moins 70 °C.

**10.** Procédé selon la revendication 9, **caractérisé en ce que** l'eau présente lors de l'ajout une température dans la plage allant de 80 °C à 100 °C.

**11.** Procédé de fabrication de compositions selon l'une quelconque des revendications 1 à 8, **caractérisé en ce que**

(a) au moins deux alcools alcoxylés différents,
(b) au moins un composant hydrophobe, choisi parmi les substances hydrophobes naturelles, naturelles modifiées et synthétiques,
(c) au moins 20 % en poids d'eau, par rapport au poids total de la composition,
(d) éventuellement au moins un émulsifiant ionique ou non ionique et
(e) éventuellement au moins un additif
sont mélangés, puis portés à une température d'au moins 70 °C.

**12.** Procédé selon l'une quelconque des revendications 9 à 11, **caractérisé en ce qu'**un mélange d'au moins 3 alcools alcoxylés est utilisé en tant que composant (a).

**13.** Utilisation de compositions selon l'une quelconque des revendications 1 à 8 ou de compositions fabriquées par un procédé selon l'une quelconque des revendications 9 à 12, pour la fabrication de cuir ou de peaux à fourrure.

**14.** Procédé de fabrication de cuir ou de peaux à fourrure utilisant des compositions selon l'une quelconque des revendications 1 à 8 ou des compositions fabriquées par un procédé selon l'une quelconque des revendications 9 à 12.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 0323069 A
- DE 19740452
- WO 0168584 A
- EP 0891430 B
- EP 0459168 A
- EP 0463401 A2
- EP 0459168 B
- WO 9841581 A


### In der Beschreibung aufgeführte Nicht-Patentliteratur


- **Herfeld.** *Bibliothek des Leders,* 1987, vol. 4, 13
- **Herfeld.** *Bibliothek des Leders,* 1987, vol. 4, 59